(19) Europäisches Patentamt
European Patent Office
Office européen des brevets

(11) EP 1 911 821 A1

(12) EUROPÄISCHE PATENTANMELDUNG

(43) Veröffentlichungstag:
**16.04.2008 Patentblatt 2008/16**

(21) Anmeldenummer: **07117446.0**

(22) Anmeldetag: **28.09.2007**

(51) Int Cl.:
***C09D 183/04*** *(2006.01)* ***C08J 3/05*** *(2006.01)*
***C09D 5/02*** *(2006.01)*

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(30) Priorität: **04.10.2006 DE 102006046957**

(71) Anmelder: **Wacker Chemie AG**
**81737 München (DE)**

(72) Erfinder: **Sandmeyer, Frank**
**84508, Burgkirchen (DE)**

(74) Vertreter: **Gössmann, Christoph Tassilo et al**
**Wacker Chemie AG**
**Zentralbereich PML**
**Hanns-Seidel-Platz 4**
**81737 München (DE)**

# (54) Partikeldispersion

(57) Verfahren zum Dispergieren von Feststoff, der bei 20°C und 1020 hPa höchstens zu 1 Gewichtsteil in 100 Gewichtsteilen Wasser löslich ist, dadurch gekennzeichnet, dass in eine wässrige Zubereitung zumindest ein (A) selbstemulgierendes Organopolysiloxan eingearbeitet wird und sodann (B) Feststoff eingearbeitet wird.

EP 1 911 821 A1

**Beschreibung**

Hydrophobe Partikel können nicht ohne weiteres homogen in Wasser dispergiert werden. Hierfür werden insbesondere Emulgatoren verwendet, die später in den Anwendungen in der Regel stören.

EP 0 609 524 B1 lehrt, dass Zubereitungen aus hydrophoben Feststoffen und Organopolysiloxanen, die Salze organischer oder anorganischer Säuren und Organopolysiloxan enthalten, welche Si-C gebundene Reste mit basischem Stickstoff aufweisen in Wasser selbstdispergierend sind. Solche Zubereitungen werden erhalten, indem man zu den Organopolysiloxanzubereitungen einen hydrophoben Feststoff zumischt. Die erhaltenen Mischungen ergeben beim Verdünnen mit Wasser homogene Dispersionen, in denen die hydrophoben Feststoffe feindispers verteilt sind. EP 0 609 524 B1 lehrt des weiteren, dass die Zubereitungen, bzw. die daraus durch Verdünnen mit Wasser zu erhaltenden wässrigen Dispersionen zur Oberflächenbehandlung verwendet werden können. Dabei werden stets nur die Zubereitungen entsprechend EP 0 609 524 B1 für die jeweilige Anwendung eingesetzt. Für die jeweils beschriebenen Anwendungen sind jedoch marktübliche Produkte vorhanden, die ihrerseits ihre Stärken haben, die nicht voll umfänglich von den Zubereitungen nach EP 0609 524 B1 nachzustellen oder zu übertreffen sind. Vielmehr haben die Zubereitungen nach EP 0609524 B1 ihre speziellen Vorzüge. Zudem ist bei den Zubereitungen gemäß EP 0 609 524 B1 der Löwenanteil der Zubereitung stets das Organopolysiloxangemisch, nicht die Partikelspezies. So ist es zum Beispiel nicht möglich mehr als 7% hydrophober pyrogener Kieselsäure vom Typ WACKER HDK® H18 in die Organopolysiloxanzubereitung (A) aus EP 0 609 524 B1 gemäß Beispiel 1 aus EP 0 609 524 B1 einzuarbeiten, da die Viskosität der Zubereitung so stark ansteigt, dass sie unter technisch relevanten Bedingungen nicht mehr verarbeitbar ist.

Die Aufgabe der vorliegenden Erfindung bestand darin den Stand der Technik zu verbessern, insbesondere ein möglichst universelles Verfahren zu entwickeln, das es gestattet hydrophobe Feststoffe in handelsübliche wässrige Produkte einzuarbeiten, ohne dass hierzu Veränderungen an der Rezeptur der Produkte notwendig sind und vor allem ohne dass hierfür Emulgatoren gebraucht werden. Dabei soll das Verfahren so breit anwendbar sein, dass die Auswahl der jeweiligen hydrophoben Feststoffe dem Formulierer weitgehend selbst überlassen bleibt.

Gegenstand der Erfindung ist ein Verfahren zum Dispergieren von Feststoff, der bei 20°C und 1020 hPa höchstens zu 1 Gewichtsteil in 100 Gewichtsteilen Wasser löslich ist, dadurch gekennzeichnet, dass in eine wässrige Zubereitung zumindest ein (A) selbstemulgierendes Organopolysiloxan eingearbeitet wird und sodann (B) Feststoff eingearbeitet wird.

"Selbstdispergierbarkeit" bedeutet im Rahmen dieser Erfindung, dass die erfindungsgemäßen Zusammensetzungen mit Wasser spontan und ohne Einsatz der üblicherweise zur Herstellung von Dispersionen aufgewendeten mechanischen Energie, durch bloßes Eingießen in Wasser und Umrühren stabile wässrige Verdünnungen ergeben.

Der Begriff "basischer Stickstoff", wie er im Rahmen dieser Erfindung mit Mengenangaben gebraucht wird, bezieht sich auf Stickstoff, berechnet als Element.

Vorzugsweise liegt neben (A) auch die Verbindung (C) Organosiliciumverbindung mit basischem Stickstoff in Mengen von 0 bis 0,5 Gewichtsprozent vor, bezogen auf das Gewicht dieser Organosiliciumverbindung.

Vorzugsweise ist das selbstemulgierende Organopolysiloxan ein

(A) Salz von organischer oder anorganischer Säure und Organopolysiloxan, welches SiC-gebundene Reste mit basischem Stickstoff in Mengen von mindestens 0,5 Gewichtsprozent basischem Stickstoff, bezogen auf das Gewicht dieses Organopolysiloxans, aufweist.

Wässrige Zubereitungen nach dem erfindungsgemäßen Verfahren sind alle Zubereitungen, in die die Polyorganosiloxanzubereitungen stabil eingearbeitet werden können.

Es sind wässrige Zubereitungen, die vorzugsweise die unten beschriebenen Eigenschaften verleihen und ihre Anwendungen, wie die Herstellung von Beschichtungsstoffen und Imprägnierungen und daraus zu erhaltenden Beschichtungen und Überzügen auf Substraten, außerdem zum Zwecke der Entschäumung, der Verlaufsförderung, Hydrophobierung, Hydrophilierung, Füllstoff-und Pigmentdispergierung, Füllstoff- und Pigmentbenetzung, Substratbenetzung, Förderung der Oberflächenglätte, Reduzierung des Haft- und Gleitwiderstandes.

Die Organopolysiloxane, durch deren Umsetzung mit organischer oder anorganischer Säure Bestandteil (A) der erfindungsgemäßen Zusammensetzung erhältlich ist, sind vorzugsweise solche der Formel

$$R_aR^1_b(OR^2)_cSiO_{\frac{4-a-b-c}{2}} \qquad (I)$$

worin

R gleich oder verschieden sein kann und Wasserstoff oder einwertige, von basischem Stickstoff freie, SiC-gebundene

organische Reste bedeutet,
$R^1$ gleich oder verschieden sein kann und einwertige, SiC-gebundene Reste mit basischem Stickstoff bedeutet,
$R^2$ gleich oder verschieden sein kann und Wasserstoffatom oder einwertige organische Reste bedeutet,
a 0, 1, 2 oder 3,
b 0, 1, 2 oder 3 und
c 0, 1, 2 oder 3 ist,
mit der Maßgabe, dass die Summe aus a, b und c kleiner oder gleich 3 ist und Rest $R^1$ in Mengen von mehr als 0,5 Gewichtsprozent basischem Stickstoff pro Organopolysiloxanmolekül vorhanden ist.

Bevorzugt handelt es sich bei Rest R um gegebenenfalls substituierte Kohlenwasserstoffreste mit 1 bis 20 Kohlenstoffatomen, wobei Kohlenwasserstoffreste mit 1 bis 8 Kohlenstoffatomen, insbesondere der Methyl- und der Isooctylrest, besonders bevorzugt sind.

Vorzugsweise ist an jedes Siliciumatom, an das ein Wasserstoffatom gebunden ist, auch ein Kohlenwasserstoffrest, insbesondere ein Methylrest, gebunden.

Beispiele für Reste R sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, Octadecylreste, wie der n-Octadecylrest; Alkenylreste, wie der Vinyl-, Allyl-, n-5-Hexenyl-, 4-Vinylcyclohexyl- und der 3-Norbornenylrest; Cycloalkylreste, wie Cyclopentyl-, Cyclohexyl-, 4-Ethylcyclohexyl-, Cycloheptylreste, Norbornylreste und Methylcyclohexylreste; Arylreste, wie der Phenyl-, Biphenylyl-, Naphthyl- und Anthryl-und Phenanthrylrest; Alkarylreste, wie o-, m-, p-Tolylreste, Xylylreste und Ethylphenylreste; Aralkylreste, wie der Benzylrest, der alpha- und der β-Phenylethylrest.

Beispiele für substituierte Kohlenwasserstoffreste als Rest R sind halogenierte Kohlenwasserstoffreste, wie der Chlormethyl-, 3-Chlorpropyl-, 3-Brompropyl-, 3,3,3-Trifluorpropyl- und 5,5,5,4,4,3,3-Heptafluorpentylrest, sowie der Chlorphenyl-, Dichlorphenyl- und Trifluortolylrest; Mercaptoalkylreste, wie der 2-Mercaptoethyl- und 3-Mercaptopropylrest; Cyanoalkylreste, wie der 2-Cyanoethyl- und 3-Cyanopropylrest; Acyloxyalkylreste, wie der 3-Acryloxypropyl- und 3-Methacryloxypropylrest; Hydroxyalkylreste, wie der Hydroxypropylrest und Reste der Formel

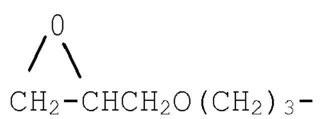

und $HOCH_2CH(OH)CH_2SCH_2CH_2$-.
Bevorzugt handelt es sich bei Rest $R^1$ um solche der Formel

$R^3{}_2NR^4$- (II),

worin $R^3$ gleich oder verschieden sein kann und Wasserstoff oder einwertiger, gegebenenfalls mit Aminogruppen substituierter Kohlenwasserstoffrest bedeutet und $R^4$ zweiwertiger Kohlenwasserstoffrest bedeutet.

Beispiele für Rest $R^3$ sind die für Rest R gegebenen Beispiele für Kohlenwasserstoffreste sowie mit Aminogruppen substituierte Kohlenwasserstoffreste, wie Aminoalkylreste, wobei der Aminoethylrest besonders bevorzugt ist.

Vorzugsweise ist an jedes Stickstoffatom in den Resten der Formel (II) mindestens ein Wasserstoffatom gebunden.

Bevorzugt handelt es sich bei Rest $R^4$ um zweiwertige Kohlenwasserstoffreste mit 1 bis 10 Kohlenstoffatomen, besonders bevorzugt 1 bis 4 Kohlenstoffatomen, insbesondere um den n-Propylenrest.

Beispiele für Rest $R^4$ sind der Methylen-, Ethylen-, Propylen-, Butylen-, Cyclohexylen-, Octadecylen-, Phenylen- und Butenylenrest.

Beispiele für Reste $R^1$ sind
$H_2N(CH_2)_3$-,
$H_2N(CH_2)_2NH(CH_2)_2$-,
$H_2N(CH_2)_2NH(CH_2)_3$-,
$H_2N(CH_2)_2$-,
$H_3CNH(CH_2)_3$-,
$C_2H_5NH(CH_2)_3$-,
$H_3CNH(CH_2)_2$-,

$C_2H_5NH\ (CH_2)_2$-,
$H_2N(CH_2)_4$-,
$H_2N(CH_2)_5$-,
$H(NHCH_2CH_2)_3$-,
$C_4H_9NH(CH_2)_2NH(CH_2)_2$-,
cyclo-$C_6H_{11}NH\ (CH_2)_3$-,
cyclo-$C_6H_{11}NH\ (CH_2)_2$-,
$(CH_3)_2N(CH_2)_3$-,
$(CH_3)_2N(CH_2)_2$-,
$(C_2H_5)_2N(CH_2)_3$- und
$(C_2H_5)_2N\ (CH_2)_2$.

Bevorzugt handelt es sich bei Rest $R^1$ um $H_2N(CH_2)_3$- und $H_2N\ (CH_2)_2NH\ (CH_2)_3$-, wobei $H_2N\ (CH_2)_2NH\ (CH_2)_3$- besonders bevorzugt ist.

Des weiteren kann es sich bei Rest $R^1$ auch um cyclische Aminreste, wie Piperidylreste, handeln.

Bevorzugt handelt es sich bei Rest $R^2$ um Wasserstoffatom und Alkylreste mit 1 bis 4 Kohlenstoffatomen, wobei der Methyl-, Ethyl- und Propylrest besonders bevorzugt sind.

Die Beispiele für Alkylreste R gelten im vollen Umfang auch für den Rest $R^2$.

Der durchschnittliche Wert für a ist 0 bis 2, vorzugsweise 0 bis 1,8.

Der durchschnittliche Wert für b ist 0,1 bis 0,6, vorzugsweise 0,15 bis 0,30.

Der durchschnittliche Wert für c ist 0 bis 0,8, vorzugsweise 0,01 bis 0,6.

Beispiele für Organopolysiloxane aus Einheiten der Formel (I) sind das Umsetzungsprodukt von Tetraethylsilikat mit N-(2-Aminoethyl) 3-aminopropyltrimethoxysilan mit einer Viskosität bei 25°C von 6 bis 7 $mm^2/s$ und einer Aminzahl von 2,15 (Siloxan i), das Umsetzungsprodukt von $\alpha,\omega$-Dihydroxydimethylpolysiloxan und N-(2-Aminoethyl)-3-aminopropyltrimethoxysilan mit einer Viskosität von 20 bis 50 $mm^2/s$ (25°C) und einer Aminzahl von 2,7 bis 3,2 (Siloxan ii) sowie das Umsetzungsprodukt von $CH_3Si\ (OC_2H_5)_{0,8}O_{1,1}$ und N- (2-Aminoethyl) -3-aminopropyltrimethoxysilan mit einer Viskosität von 60 $mm^2/s$ (25°C) und einer Aminzahl von 2,15 (Siloxan iii), wobei (Siloxan ii) und (Siloxan iii) bevorzugt und (Siloxan ii) besonders bevorzugt sind und die Aminzahl der Anzahl der ml 1-n-HCl, die zum Neutralisieren von 1 g Substanz erforderlich sind, entspricht.

Vorzugsweise haben die Organopolysiloxane aus Einheiten der Formel (I) eine Viskosität von 6 bis 60 $mm^2/s$, bezogen auf 25°C.

Organopolysiloxane aus Einheiten der Formel (I) können in bekannter Weise, beispielsweise durch Äquilibrieren bzw. Kondensieren von aminofunktionellen Silanen mit Organopolysiloxanen, die frei von basischem Stickstoff sind, hergestellt werden.

Die organischen oder anorganischen Säuren, die zur Herstellung von Bestandteil (A) der erfindungsgemäßen Zusammensetzung verwendet werden, können die gleichen sein, die auch bisher zur Herstellung von Salzen von organischer oder anorganischer Säure und Organopolysiloxan mit basischen Stickstoff aufweisenden, SiC-gebundenen Resten eingesetzt werden konnten. Beispiele für derartige Säuren sind vorzugsweise HCl, $H_2SO_4$, Essigsäure, Propionsäure und Diethylhydrogenphosphat, wobei Essigsäure und Propionsäure bevorzugt und Essigsäure besonders bevorzugt sind.

Verbindungen, die als Komponente (A) in der erfindungsgemäßen Zusammensetzung eingesetzt werden können, sind bereits bekannt. Hierzu sei beispielsweise auf US 4,661,551 verwiesen.

Bei dem als Komponente (A) eingesetzten Organopolysiloxansalz kann es sich um eine einzelne Art dieses Salzes wie auch um ein Gemisch aus mindestens zwei Arten eines solchen Salzes handeln.

Bei den erfindungsgemäß eingesetzten hydrophoben Feststoffen (B), das heißt Feststoffen, die bei 20°C und 1020 hPa höchstens zu einem Gewichtsteil in 100 Gewichtsteilen Wasser löslich sind, handelt es sich bevorzugt um Füllstoffe, Pigmente, Biozide und ultraviolettes Licht absorbierende Feststoffe, vorzugsweise ausgenommen bei 20°C und 1020 hPa feste Organosiliciumverbindungen, die sich unter diesen Bedingungen zu mehr als 50 Gewichtsteilen in 100 Gewichtsteilen (A) gegebenenfalls im Gemisch mit (C) lösen.

Beispiele für hydrophobe Füllstoffe sind vorzugsweise nicht verstärkende Füllstoffe, also Füllstoffe mit einer BET-Oberfläche von bis zu 50 $m^2/g$, wie Quarz, Diatomeenerde, Calciumsilikat, Zirkaniumsilikat, Zeolithe, Montmorrillonite, wie Bentonite, Metalloxidpulver, wie Aluminium-, Titan-, Eisen-, oder Zinkoxide bzw. deren Mischoxide, Bariumsulfat, Calciumcarbonat, Siliciumnitrid, Siliciumcarbid, Bornitrid, Glas- und Kunststoffpulver; vorzugsweise verstärkende Füllstoffe, also Füllstoffe mit einer BET-Oberfläche von mehr als 50 $m^2/g$, wie pyrogen hergestellte Kieselsäure, gefällte Kieselsäure, Ruß, wie Furnace- und Acetylenruß und Silicium-Aluminium-Mischoxide großer BET-Oberfläche; faserförmige Füllstoffe, wie Asbest sowie Kunststoffasern. Die genannten Füllstoffe können hydrophobiert sein, beispielsweise durch die Behandlung mit Organosilanen bzw. -siloxanen oder durch Veretherung von Hydroxylgruppen zu Alkoxygruppen.

Beispiele für Pigmente sind Erdpigmente, wie vorzugsweise Kreide, Ocker, Umbra, Grünerde, Mineralpigmente,

wie Titandioxid, Chrom-gelb, Mennige, Zinkgelb, Zinkgrün, Cadmiumrot, Kobaltblau, organische Pigmente, wie Sepia, Kasseler Braun, Indigo, Azo-Pigmente, Antrachinoide-, Indigoide-, Dioxazin-, Chinacridon-, Phthalocyanin-, Isoindolinon- und Alkaliblau-Pigmente, wobei viele der anorganischen Pigmente auch als Füllstoffe fungieren und umgekehrt.

Beispiele für hydrophobe Biozide sind, Fungizide, Insektizide, Herbizide und Algizide, wie Benzimidazolderivate.

Beispiele für ultraviolettes Licht absorbierende Feststoffe sind Benztriazol, Tolyltriazol und transparentes Eisenpigment.

Bevorzugt enthält die erfindungsgemäße Zusammensetzung als Feststoff (B) hydrophobe pyrogenehochdisperse Kieselsäure mit einer Oberfläche von etwa 140 $m^2$/g, welche durch Flammenhydrolyse flüchtiger Siliciumverbindungen und anschließender Hydrophobierung mit Organosilanen hergestellt werden kann.

Vorzugsweise enthalten die erfindungsgemäßen Zusammensetzungen hydrophoben Feststoff (B) in Mengen von 0,1 bis 15 Gewichtsteilen, besonders bevorzugt von 0,5 bis 2 Gewichtsteilen, je Gewichtsteil Bestandteil (A).

Es kann eine Art von Feststoff (B) wie auch ein Gemisch von mindestens zwei verschiedenen Arten derartiger Feststoffe eingesetzt werden.

Bei der gegebenenfalls eingesetzten Organosiliciumverbindung (C) handelt es sich vorzugsweise um solche aus Einheiten der Formel

$$R^5_d(OR^6)_eSiO_{\frac{4-d-e}{2}} \qquad (III),$$

worin

$R^5$ gleich oder verschieden sein kann und Wasserstoff oder einwertige, SiC-gebundene organische Reste bedeutet,

$R^6$ gleich oder verschieden sein kann und Wasserstoffatom oder einwertige organische Reste bedeutet,

d 0, 1, 2, 3 oder 4 und

e 0, 1, 2, 3 oder 4 ist,

mit der Maßgabe, dass die Summe aus d und e kleiner oder gleich 4 ist und der Gehalt an basischem Stickstoff 0 bis 0,5 Gewichtsprozent, bezogen auf das Gewicht der jeweiligen Organosiliciumverbindung, beträgt.

Beispiele für Rest $R^5$ sind die für Rest R angegebenen Beispiele sowie mit Aminogruppen substituierte Kohlenwasserstoffreste, wobei Kohlenwasserstoffreste mit 1 bis 8 Kohlenstoffatomen bevorzugt und der Methyl- und Isooctylrest besonders bevorzugt sind.

Beispiele für Rest $R^6$ sind die für $R^2$ angegebenen Beispiele, wobei der Methyl-, Ethyl- und Propylrest bevorzugt und der Methyl- und Ethylrest besonders bevorzugt sind.

Bei der Organosiliciumverbindung aus Einheiten der Formel (III) kann es sich um Silane handeln, d.h. die Summe aus d und e ist gleich 4.

Bei den Organosiliciumverbindungen aus Einheiten der Formel (III) kann es sich auch um Organopolysiloxane handeln, d.h. die Summe aus d und e ist kleiner oder gleich 3.

Beispiele für Silane der Formel (III) sind i-Octyltrimethoxysilan und i-Octyltriethoxysilan.

Beispiele für Organopolysiloxane aus Einheiten der Formel (III) sind Methylethoxypolysiloxane, Dimethylpolysiloxane und i-Octylmethoxypolysiloxane.

Vorzugsweise haben die Organopolysiloxane aus Einheiten der Formel (III) eine Viskosität von 5 bis 2000 $mm^2$/s, besonders bevorzugt 10 bis 500 $mm^2$/s, jeweils bezogen auf 25°C.

Bei der gegebenenfalls eingesetzten Organosiliciumverbindung (C) handelt es sich besonders bevorzugt um Silane und niedermolekulare Siloxane, insbesondere um Silane.

Verfahren zur Herstellung der Organosiliciumverbindungen aus Einheiten der Formel(III) sind vielfach bekannt.

Falls zur Herstellung der erfindungsgemäßen Zusammensetzung Organosiliciumverbindung (C) verwendet wird, wird diese in Mengen von vorzugsweise 0,5 bis 15 Gewichtsteilen, besonders bevorzugt 1 bis 3 Gewichtsteilen, je Gewichtsteil Komponente (A), eingesetzt.

Bevorzugt enthalten die erfindungsgemäßen Zusammensetzungen Komponente (C).

Bei der gegebenenfalls eingesetzten Organosiliciumverbindung (C) kann es sich um eine Art wie auch um ein Gemisch aus mindestens zwei Arten einer derartigen Organosiliciumverbindung handeln.

Die erfindungsgemäßen Zusammensetzungen können weitere Komponenten, wie beispielsweise Konservierungsmittel, Dispergiermittel und organisches Lösungsmittel, enthalten.

Vorzugsweise sind die erfindungsgemäßen Zusammensetzungen jedoch frei von organischem Lösungsmittel oder enthalten organisches Lösungsmittel in Mengen von höchstens 10 Gewichtsprozent, bezogen auf das Gesamtgewicht an Komponente (A) und gegebenenfalls eingesetzter Komponente (C).

Zugleich weisen die Emulgatoren, von denen die Zusammensetzungen nach dem erfindungsgemäßen Verfahren vorzugsweise im wesentlichen frei sind, zumeist eine Löslichkeit in Wasser bei 20 °C und dem Druck der umgebenden Atmosphäre, also 900 bis 1100 hPa, homogen oder in Micellen-Form von größer 1 Gew.-% auf.

Die Zusammensetzungen nach dem erfindungsgemäßen Verfahren können solche oberflächenaktive Substanzen bis zu einer maximalen Konzentration von kleiner als 0,1 mal, bevorzugt kleiner als 0,01 mal, besonders bevorzugt kleiner als 0,001 mal, insbesondere kleiner als 0,0001 mal der kritischen Micellkonzentration dieser oberflächenaktiven Stoffe in der Wasserphase enthalten; dies entspricht einer Konzentration dieser oberflächenaktiven Substanzen, bezogen auf das Gesamtgewicht der erfindungsgemäßen Emulsion von kleiner 10 Gew.-%, bevorzugt kleiner 2 Gew.-%, besonders bevorzugt kleiner 1 Gew.-%, insbesondere 0 Gew.-%.

Das Verfahren ist prinzipiell auf alle Zubereitungen anwendbar, in die die Polyorganosiloxanzubereitungen stabil eingearbeitet werden können. Aufgrund der Siloxaneigenschaften und der Eigenschaften, die von hydrophoben Festkörpern zu erwarten sind, sind die Hauptanwendungsfelder sicherlich in den Bereichen angesiedelt, in denen eine leistungsfähige Hydrophobierung eine Rolle spielt. Dazu gehören vordergründig die Hydrophobierung von mineralischen Baustoffen einschließlich Fassaden, Straßen und Brücken, wie z.B. Dachziegel, Ziegelsteinen, bewehrtem oder unbewehrtem Beton, Gips, Schlackensteinen und Kalksandsteinen und Holz sowie die hydrophobierende Behandlung von Textilien, Leder, Metallen zum Beispiel zum Korrosionsschutz, Papier und Pappe. Außerdem bietet sich die Anwendung des erfindungsgemäßen Verfahrens insbesondere zur Herstellung von mit Wasser verdünnbaren Zubereitungen an, wie Anstrichfarben, Verputzen, Polituren, etc. an.

Außer zum Zwecke der Hydrohpbierung kann das erfindungsgemäße Verfahren auch auf Zubereitungen angewendet werden, die verwendet werden zum Zwecke des Korrosionsschutzes auf Metallen und zur Manipulation von weiteren Eigenschaften, wie z.B.:

- Steuerung der elektrischen Leitfähigkeit und des elektrischen Widerstandes
- Steuerung der Verlaufseigenschaften einer Zubereitung
- Steuerung des Glanzes eines feuchten oder gehärteten Filmes oder eines Objektes
- Erhöhung der Bewitterungsbeständigkeit
- Erhöhung der Chemikalienresistenz
- Erhöhung der Farbtonstabilität
- Reduzierung der Kreidungsneigung
- Reduzierung oder Erhöhung der Haft- und Gleitreibung
- Stabilisierung oder Destabilisierung von Schaum
- Verbesserung der Haftung,
- Steuerung des Füllstoff- und Pigmentnetz- und - dispergierverhaltens,
- Steuerung der rheologischen Eigenschaften
- Steuerung der mechanischen Eigenschaften, wie z.B. Flexibilität, Kratzfestigkeit, Elastizität, Dehnbarkeit, Biegefähigkeit, Reißverhalten, Rückprallverhalten, Härte, Dichte, Weiterreißfestigkeit, Druckverformungsrest, Verhalten bei verschiedenen Temperaturen, Ausdehnungskoeffizient, Abriebfestigkeit sowie weiterer Eigenschaften wie der Wärmeleitfähigkeit, Brennbarkeit, Gasdurchlässigkeit, Beständigkeit gegen Wasserdampf, Heißluft, Chemikalien, Bewitterung und Strahlung, der Sterilisierbarkeit
- Steuerung der elektrischen Eigenschaften, wie z.B. dielektrischer Verlustfaktor, Durchschlagfestigkeit, Dielektrizitätskonstante, Kriechstromfestigkeit, Lichtbogenbeständigkeit, Oberflächenwiderstand, spezifischer Durchschlagswiderstand,

Beispiele für Anwendungen, für die das erfindungsgemäße Verfahren eingesetzt werden kann, um die oben bezeichneten Eigenschaften zu manipulieren, sind die Herstellung von Beschichtungsstoffen und Imprägnierungen und daraus zu erhaltenden Beschichtungen und Überzügen auf Substraten, wie vorzugsweise Metall, Glas, Holz, mineralisches Substrat, Kunst-und Naturfasern zur Herstellung von Textilien, Teppichen, Bodenbelägen, oder sonstigen aus Fasern herstellbaren Gütern, Leder, Kunststoffe wie Folien, Formteilen, außerdem zum Zwecke der Entschäumung, der Verlaufsförderung, Hydrophobierung, Hydrophilierung, Füllstoff- und Pigmentdispergierung, Füllstoff- und Pigmentbenetzung, Substratbenetzung, Förderung der Oberflächenglätte, Reduzierung des Haft- und Gleitwiderstandes.

Das erfindungsgemäße Verfahren kann auf Elastomermassen angewendet werden. Hierbei können die Anwendungsziele die Verstärkung oder die Verbesserung anderer Gebrauchseigenschaften wie der Steuerung der Transparenz, der Hitzebeständigkeit, der Vergilbungsneigung, der Bewitterungsbeständigkeit sein.

Gemäß dem erfindungsgemäßen Verfahren ist es möglich das Verhältnis von Partikel zu Organopolysiloxanzusammensetzung auch so zu gestalten, dass in den Zielzubereitungen wesentlich mehr hydrophobe Partikel eingebracht werden, als Organopolysiloxanzubereitung. Dies zeigen die Beispiele der vorliegenden Beschreibung.

### Beispiel 1

A Herstellung von Organopolysiloxanen mit basischem Stickstoff (Siloxan A)

In einen mit Rührer, Tropftrichter und Rückflußkühler ausgestatteten 1-1-Dreihalskolben werden unter Rühren zu einem Gemisch aus 0,2 g KOH in 4 g Methanol und 500 $_g$ eines $\alpha,\omega$-Dihydroxydimethylpolysiloxans mit einem durchschnittlichen Molekulargewicht von etwa 4000 g/Mol 150 g N-(2-Aminoethyl)-3-aminopropyltrimethoxysilan gegeben und das so erhaltene Gemisch 6 Stunden unter Rückfluß zum Sieden erhitzt. Anschließend wird auf 30°C abgekühlt und 2,5 ml 10%igeSalzsäure zugegeben. Durch Erhitzen auf bis zu 140°C wird schließlich das Methanol abdestilliert und das so erhaltene Organopolysiloxan durch Filtrieren von KCl befreit. Das erhaltene Organopolysiloxan hat eine Viskosität/Molgewicht von 50 $mm^2/s$ und enthält 2,9 % basischen Stickstoff, bezogen auf sein Gewicht.

20 g des oben unter A hergestellten Aminosiloxans, 3 g Essigsäure, 47 g i-Octyltrimethoxysilan und 30 g hydrophobe pyrogene hochdisperse Kieselsäure (käuflich erhältlich unter der Bezeichnung HDK H 2000 bei der Wacker-Chemie GmbH) werden miteinander vermischt, wobei sich eine homogene Mischung mit einem leichten Tyndall-Effekt ergibt. Die so erhaltene Mischung ist beim Einbringen in Wasser spontan selbstdispergierend, wobei sich die hydrophobe Kieselsäure feinstdispers in Wasser verteilt. Die so erhaltene 10 %ige wäßrige Verdünnung ist bei Raumtemperatur für einen Zeitraum von über 6 Monaten stabil und zeigt im Transmissionselektronenmikroskop eine Teilchengröße der hydrophoben Kieselsäure von ca. 10 bis 20 nm.

### Beispiel 2

B Herstellung von Organopolysiloxanen mit basischem Stickstoff (Siloxan B)

In einen mit Rührer, Tropftrichter und Rückflußkühler ausgestatteten 1-1-Dreihalskolben werden unter Rühren zu einem Gemisch aus 0,2 g KOH in 4 g Methanol und 500 g eines Organopolysiloxans der Summenformel $CH_3Si(OC_2H_5)_{0,8}O_{1,1}$

mit einem durchschnittlichen Molekulargewicht von etwa 600 g/Mol und einer Viskosität von ca. 20 $mm^2/s$ 150 g N-(2-Aminoethyl)-3-aminopropyltrimethoxysilan gegeben und das so erhaltene Gemisch 6 Stunden unter Rückfluß zum Sieden erhitzt. Anschließend wird auf 30°C abgekühlt und 2,5 ml 10%ige Salzsäure zugegeben. Durch Erhitzen auf bis zu 140°C wird schließlich das Methanol abdestilliert und das so erhaltene Organopolysiloxan durch Filtrieren von KCl befreit. Das erhaltene Organopolysiloxan hat eine Viskosität von 60 $mm^2/s$ und ein Molgewicht von etwa 1800 und enthält 2,9 % basischen Stickstoff, bezogen auf sein Gewicht.

25 g des oben unter B hergestellten Aminosiloxans, 5 g Propionsäure, 65 g Propyltrimethoxysilan und 5 g eines UV-Lichtschutzmittels, welches als UV-Absorber Benztriazol enthält, (käuflich erhältlich unter der Bezeichnung "Tinuvin 320" bei der Ciba-Geigy) werden miteinander vermischt, wobei sich eine homogene Mischung mit einem leichten Tyndall-Effekt ergibt. Die so erhaltene Mischung ist beim Einbringen in Wasser spontan selbstdispergierend, wobei sich das hydrophobe UV-Lichtschutzmittel feinstdispers in Wasser verteilt.
Die so erhaltene 10 %ige wäßrige Verdünnung ist bei Raumtemperatur für einen Zeitraum von über 6 Monaten stabil und zeigt im Transmissionselektronenmikroskop eine Teilchengröße des UV-Lichtschutzmittels von ca. 10 bis 50 nm.

### Beispiel 3

Einbringung hydrophober pyrogener Kieselsäure in eine wässrige Zubereitung eines Polyacrylates unter Verwendung des Organopolysiloxangemisches (A)

Zu 135 g einer 20 gewichtsprozentigen wässrigen Dispersion eines carboxyfunktionellen und mit Ammoniak neutralisierten Polyacrylates, das so eingestellt ist, dass es selbstemulgierend ist, wobei zur Erhöhung der Dispersionsstabilität 0,2 Gewichtsprozent bezogen auf Festgehalt Natriumdodecylsulfonat zugegeben wurden und die Partikelgröße des Polyacrylates auf unter 100 nm eingestellt wurde, wurden 15 g des Organopolysiloxangemisches (A) gegeben und durch umrühren mit einem Flügelrührer homogen verteilt. Danach wurden 9,9 g pyrogene Kieselsäure (WACKER HDK® H 18) unter Rühren am Dissolver (2000 U/min) zugegeben. Nach vollständiger HDK Zugabe wurde weitere 5 min gerührt. Man erhielt eine Paste, die sich durch Wasserzugabe zu einer leicht beweglichen wässrigen Zubereitung verdünnen ließ, in der die pyrogene Kieselsäure homogen verteilt und feindispers vorliegt.

**Vergleichsbeispiel 1**

Einbringung hydrophober pyrogener Kieselsäure in eine wässrige Zubereitung eines Polyacrylates unter Verwendung des Organopolysiloxangemisches (A)

Zu 100 g einer 20 gewichtsprozentigen wässrigen Dispersion eines carboxyfunktionellen und mit Ammoniak neutralisierten Polyacrylates, das so eingestellt ist, dass es selbstemulgierend ist, wobei zur Erhöhung der Dispersionsstabilität 0,2 Gewichtsprozent, bezogen auf den Festgehalt, Natriumdodecylsulfonat zugegeben wurden und die Partikelgröße des Polyacrylates auf unter 100 nm eingestellt wurde, wurden 6,6 g pyrogene Kieselsäure (WACKER HDK® H 18) unter Rühren am Dissolver (2000 U/min) zugegeben. Nach vollständiger HDK Zugabe wurde weitere 15 min versucht zu dispergieren, jedoch ohne Erfolg, die pyrogene Kieselsäure lässt sich nicht in die wässrige Phase einarbeiten.

**Beispiel 4**

Einbringung hydrophober pyrogener Kieselsäure in eine wässrige Zubereitung eines Polyacrylates unter Verwendung des Organopolysiloxangemisches (A):

Zu 135 g einer 20 gewichtsprozentigen wässrigen Dispersion eines carboxyfunktionellen und mit Ammoniak neutralisierten Polyacrylates, das so eingestellt ist, dass es selbstemulgierend ist, wobei zur Erhöhung der Dispersionsstabilität 0,2 Gewichtsprozent, bezogen auf den Festgehalt, Natriumdodecylsulfonat zugegeben wurden und die Partikelgröße des Polyacrylates auf unter 100 nm eingestellt wurde, wurden 2 g des Organopolysiloxangemisches (A) gegeben und durch Umrühren mit einem Flügelrührer homogen verteilt. Danach wurden 12,0 g pyrogene Kieselsäure (WACKER HDK® H 18) unter Rühren am Dissolver (2000 U/min) zugegeben. Nach vollständiger HDK Zugabe wurde weitere 5 min gerührt. Man erhielt eine Paste, die sich durch Wasserzugabe zu einer leicht beweglichen wässrigen Zubereitung verdünnen ließ, in der die pyrogene Kieselsäure homogen verteilt und feindispers vorliegt.

**Vergleichsbeispiel 2**

Eindispergieren einer maximalen Menge von pyrogener Kieselsäure WACKER HDK® H 18 in Organopolysiloxangemisch (A):

Zu 20 g Polyorganosiloxangemisch (A) werden 1,2 g pyrogene Kieselsäure WACKER HDK® H 18 zugegeben und mit einem Dissolver (2000 U/min) homogen eindispergiert. Es entsteht eine Paste, die keine weitere Aufnahme von pyrogener Kieselsäure mehr zulässt. Eine auch nur annähernd ähnlich große Menge WACKER HDK® H 18, gerechnet auf die eingesetzte Menge an Organopolysiloxan (A) wie in Beispiel 4, kann auf diese Weise nicht in diese Zubereitung eingebracht werden.

## Patentansprüche

1. Verfahren zum Dispergieren von Feststoff, der bei 20°C und 1020 hPa höchstens zu 1 Gewichtsteil in 100 Gewichtsteilen Wasser löslich ist, **dadurch gekennzeichnet, dass** in eine wässrige Zubereitung zumindest ein (A) selbstemulgierendes Organopolysiloxan eingearbeitet wird und sodann (B) Feststoff eingearbeitet wird.

2. Verfahren zum Dispergieren von Feststoff in Wasser nach Anspruch 1, **dadurch gekennzeichnet, dass** und neben (A) (C) Organosiliciumverbindung mit basischem Stickstoff in Mengen von 0 bis 0,5 Gewichtsprozent, bezogen auf das Gewicht dieser Organosiliciumverbindung eingearbeitet wird.

3. Verfahren zum Dispergieren von Feststoff in Wasser nach Anspruch 1 oder 2, **dadurch gekennzeichnet, dass** das selbstemulgierende Organopolysiloxan ein (A) Salz von organischer oder anorganischer Säure und Organopolysiloxan, welches SiC-gebundene Reste mit basischem Stickstoff in Mengen von mindestens 0,5 Gewichtsprozent basischem Stickstoff, bezogen auf das Gewicht dieses Organopolysiloxans, aufweist.

4. Verfahren zum Dispergieren von Feststoff in Wasser nach einem oder mehreren der Ansprüche 1 bis 3, **dadurch gekennzeichnet, dass** die Organopolysiloxane, durch deren Umsetzung mit organischer oder anorganischer Säure Bestandteil (A) erhältlich ist, solche der Formel

$$R_aR^1_b(OR^2)_cSiO_{\frac{4-a-b-c}{2}} \quad (I)$$

sind, worin
R gleich oder verschieden sein kann und Wasserstoff oder einwertige, von basischem Stickstoff freie, SiC-gebundene organische Reste bedeutet,
$R^1$ gleich oder verschieden sein kann und einwertige, SiC-gebundene Reste mit basischem Stickstoff bedeutet,
$R^2$ gleich oder verschieden sein kann und Wasserstoffatom oder einwertige organische Reste bedeutet,
a 0, 1, 2 oder 3 ,
b 0, 1, 2 oder 3 und
c 0, 1, 2 oder 3 ist.
mit der Maßgabe, daß die Summe aus a, b und c kleiner oder gleich 3 ist und Rest $R^1$ in Mengen von mehr als 0,5 Gewichtsprozent basischem Stickstoff pro Organopolysiloxanmolekül vorhanden ist.

**5.** Verfahren zum Dispergieren von Feststoff in Wasser nach einem oder mehreren der Ansprüche 1 bis 4, **dadurch gekennzeichnet, dass** die Organosiliciumverbindung (C) solche aus Einheiten der Formel

$$R^5_d(OR^6)_eSiO_{\frac{4-a-b-c}{2}} \quad (III)$$

sind, worin
$R^5$ gleich oder verschieden sein kann und Wasserstoff oder einwertige, SiC-gebundene organische Reste bedeutet,
$R^6$ gleich oder verschieden sein kann und Wasserstoffatom oder einwertige organische Reste bedeutet,
d 0, 1, 2, 3 oder 4 und
e 0, 1, 2, 3 oder 4 ist,
mit der Maßgabe,dass die Summe aus d und e kleiner oder gleich 4 ist und der Gehalt an basischem Stickstoff 0 bis 0,5 Gewichtsprozent, bezogen auf das Gewicht der jeweiligen Organosiliciumverbindung, beträgt.

**6.** Verfahren zum Dispergieren von Feststoff in Wasser nach einem oder mehreren der Ansprüche 1 bis 5, **dadurch gekennzeichnet, dass** es sich bei dem Feststoff (B) um solche, ausgewählt aus der Gruppe bestehend aus Füllstoffe, Pigmente, Biozide und ultraviolettes Licht absorbierende Feststoffe, handelt.

**7.** Verfahren zum Dispergieren von Feststoff in Wasser nach einem oder mehreren der Ansprüche 1 bis 6, **dadurch gekennzeichnet, dass** die Dispersion zum Beschichten oder Imprägnieren verwendet wird.

Europäisches Patentamt

# EUROPÄISCHER RECHERCHENBERICHT

Nummer der Anmeldung
EP 07 11 7446

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 18 16 392 A1 (GEN ELECTRIC) 21. August 1969 (1969-08-21) * Ansprüche; Beispiele 3,12,24,27 * | 1-7 | INV. C09D183/04 C08J3/05 C09D5/02 |
| X | DE 17 69 287 A1 (SIMONIZ COMPANY) 1. Oktober 1970 (1970-10-01) * Seite 1, Absatz 1 - Seite 14, Absatz 1; Ansprüche 8,9; Beispiele 4.6,13-15 * | 1-7 | |
| A,D | EP 0 609 524 B1 (WACKER CHEMIE GMBH [DE]) 13. November 1996 (1996-11-13) * Ansprüche; Beispiele * | 1-7 | |
| A | DE 36 13 384 C1 (WACKER CHEMIE GMBH) 7. Januar 1988 (1988-01-07) * Seite 5, Zeilen 39-63; Ansprüche; Beispiele * | 1-7 | |
| | | | RECHERCHIERTE SACHGEBIETE (IPC) |
| | | | C09D C08J |

Der vorliegende Recherchenbericht wurde für alle Patentansprüche erstellt

2

| Recherchenort | Abschlußdatum der Recherche | Prüfer |
|---|---|---|
| München | 28. Januar 2008 | Kolitz, Roderich |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 07 11 7446

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-01-2008

| Im Recherchenbericht angeführtes Patentdokument | Datum der Veröffentlichung | Mitglied(er) der Patentfamilie | Datum der Veröffentlichung |
|---|---|---|---|
| DE 1816392 A1 | 21-08-1969 | FR 1598723 A | 06-07-1970 |
| | | GB 1254321 A | 17-11-1971 |
| | | JP 50001356 B | 17-01-1975 |
| | | NL 6818330 A | 30-06-1969 |
| DE 1769287 A1 | 01-10-1970 | BE 714391 A | 16-09-1968 |
| | | FR 1565807 A | 02-05-1969 |
| | | GB 1185957 A | 02-04-1970 |
| | | LU 55991 A1 | 14-11-1969 |
| | | NL 6806047 A | 04-11-1968 |
| EP 0609524 B1 | 13-11-1996 | AT 145231 T | 15-11-1996 |
| | | BR 9304988 A | 05-07-1994 |
| | | CA 2102949 A1 | 11-06-1994 |
| | | CN 1089967 A | 27-07-1994 |
| | | DE 4241727 A1 | 16-06-1994 |
| | | EP 0609524 A1 | 10-08-1994 |
| | | ES 2093906 T3 | 01-01-1997 |
| | | FI 934961 A | 11-06-1994 |
| | | HU 66798 A2 | 28-12-1994 |
| | | JP 6279682 A | 04-10-1994 |
| | | MX 9307808 A1 | 30-06-1994 |
| | | NO 934498 A | 13-06-1994 |
| | | US 5661196 A | 26-08-1997 |
| DE 3613384 C1 | 07-01-1988 | EP 0242798 A2 | 28-10-1987 |
| | | JP 1838875 C | 25-04-1994 |
| | | JP 5048787 B | 22-07-1993 |
| | | JP 62256862 A | 09-11-1987 |
| | | US 4757106 A | 12-07-1988 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0609524 B1
- US 4661551 A